# EUROPEAN PATENT SPECIFICATION

(11) **EP 3 331 502 B1**
(45) Date of publication and mention of the grant of the patent: **26.05.2021**
(21) Application number: 15760312.7
(22) Date of filing: 07.08.2015
(51) Int. Cl.: A61K 9/20, A61K 31/445

(54) **CONTROLLED RELEASE PROPIVERINE FORMULATIONS**
PROPIVERIN-FORMULIERUNGEN MIT KONTROLLIERTER FREISETZUNG
FORMULATIONS DE PROPIVÉRINE À LIBÉRATION CONTRÔLÉE

(43) Date of publication of application: 13.06.2018
(73) Proprietor: Santa Farma Ilaç Sanayi A.S., 34382 Istanbul (TR)
(72) Inventor: BAS, Kaan, 34091 Istanbul (TR); OZTUNA, Banu, 34382 Sisli/Istanbul (TR); HIRA, Derya, 34091 Istanbul (TR); KANIK, Bayram, 34091 Istanbul (TR); AKSOY, Ediz, 34091 Istanbul (TR); UNVER, Levent, 34091 Istanbul (TR); ONER, Levent, 06610 Cankaya/Ankara (TR)
(74) Representative: Bulut, Pinar
(86) International application number: PCT/TR2015/050060
(87) International publication number: WO 2017/026950

(56) References cited:
- EP-A1- 2 090 297
- WO-A1-03/030869
- WO-A1-2009/129282
- US-A1- 2006 204 576
- US-A1- 2012 244 221

## Description

### Field of the Invention

The present invention relates to controlled release pharmaceutical compositions for oral administration comprising propiverine or a pharmaceutically acceptable salt thereof. More specifically, the present invention relates to a pharmaceutical composition for oral administration exhibiting a biphasic in vitro release of the active ingredient.

### Background of the Invention

Propiverine (1-methyl-4-piperidyl diphenylpropoxyacetate), a benzylic acid derivative as shown in Formula I, is one the most frequently prescribed antimuscarinic drugs used for the treatment of overactive bladder and urinary incontinence. Comparable drugs which are available in the market are oxybutynin, tolterodine tartrate and trospium chloride.

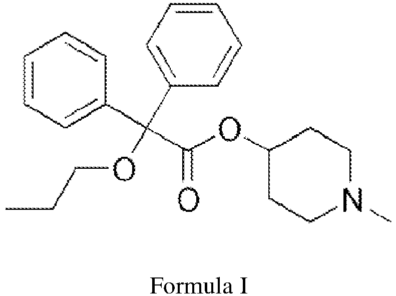

An official monograph for propiverine hydrochloride is available in Japanese Pharmacopoeia XVI (2011).

Propiverine is a BCS (Biopharmaceutical Classification System) Class III drug, which exhibits low permeability and high solubility.

Propiverine in the form of its hydrochloride is currently marketed under the trade names Mictonorm® / Detrunorm®/ Proprinorm® by Apogepha Arzneimittel GmbH in two solid dosage forms, i.e.:
1) Immediate-release (IR) tablets containing 15 mg of propiverine hydrochloride (equivalent to 13.64 mg propiverine); the usual dose is one tablet to be taken 2-3 times a day.
2) Modified-release (MR) capsules containing 30 mg or 45 mg of propiverine hydrochloride pellets (equivalent to 27.28 mg or 40.92 mg of propiverine, respectively); the usual dose is one capsule to be taken once a day.

Propiverine is nearly completely absorbed from the gastrointestinal (GI) tract. It undergoes extensive first-pass metabolism. Effects on urinary bladder smooth muscle cells are due to the parent compound and three active metabolites as well, which are rapidly excreted into the urine. After oral administration propiverine is rapidly absorbed from the GI tract with maximal plasma concentrations reached after 2.3 hours, whereas for MR capsules Cₘₐₓ is reached after 9.5-10 hours. The mean absolute bioavailability of the IR tablets is 40.5% versus 60.8±17.3% and 59.5±23.3% for the 30 mg and 45 mg MR capsules, respectively.

Food does not influence the pharmacokinetics of propiverine. The bioavailability of propiverine after the meal was 99% compared to the fasting conditions. The Cmax values for the main metabolite propiverine-N-oxide were slightly increased by food (f= 1.26), whereas the extent of absorption was unchanged. Propiverine-N-oxide showed for all pharmacokinetic parameters 90% confidence intervals within the acceptance ranges. An adjustment of dose in relation to food intake is not required (Summary of Product Characteristics of Detrunorm XL 30 mg Modified Release Capsules, last updated 23 September 2014).

Propiverine is extensively metabolized by intestinal and hepatic enzymes. The primary metabolic route involves the oxidation of the piperidyl-N and leads to the formation of the much less active propiverine-N-oxide, the plasma concentration of which greatly exceeds that of the parent substance. Four metabolites were identified in the urine; two of them a pharmacologically active and may contribute to the therapeutic efficacy of the IR tablets.

Pharmacokinetic parameters of propiverine following oral administration of 10-45 mg of propiverine hydrochloride are linearly related to dose.

The prior art includes various number of patents/patent applications relating to propiverine, modified-release pharmaceutical formulations and combination formulations thereof.

The basic patent for propiverine is DD 106643 (Starke C, Thomas G, and Friese J).

The International patent application WO 03/030869 (Apogepha Arzneimittel Gmbh) provides a prolonged-release pharmaceutical composition comprising 4mg-60mg of propiverine and/or one or several acceptable salts thereof, where the composition is adapted for once-a-day oral administration and has specific *in vitro* release characteristics measured in 750 ml of 0.1 N hydrochloric acid during the first hour and subsequently measured in 750 ml USP buffer at pH=5.8 using a Ph. Eur. basket method at 100 rpm and 37° C.

The International patent application WO 2006/046560 A1 (Taiho Pharmaceutical) provides an oral preparation that ensures good sensation and patient compliance resulting from mixing of propiverine hydrochloride, a surfactant, a pH adjusting agent and water having its pH value adjusted between 6.5 and 8.0, and subsequently granulating the suspension together with common additives for preparation.

WO 2009/129282 A1 (Eurand) discloses a pharmaceutical composition comprising a plurality of controlled-release particles, wherein a core comprising a weakly basic drug (e.g. propiverine as given in Examples 5-7), an alkaline-buffer layer disposed over the core, and a controlled-release coating comprising a water-insoluble polymer disposed over the alkaline-buffer layer.

CN 102579404 A (Guangzhou Coredes) discloses a sustained-release capsule containing micropills having uniform particle sizes wherein propiverine hydrochloride account for 75-97% and sustained-release coating layers account for 3-25% by weight percentage of the total weight.

WO 2012/154892 A1 (Theravida) provides a pharmaceutical composition comprising therapeutically effective amount of extended-release propiverine, or an acceptable salt thereof, and a therapeutically effective amount of pilocarpine (a drug for the treatment of dry mouth) or an acceptable salt thereof.

US 5,695,781 and US 6,083,532 (Hallmark Pharmaceuticals, Inc., Duramed Pharmaceuticals, Inc.) disclose a three-component release rate controlling matrix composition that includes a pH dependent gelling polymer such as alginate, a pH independent polymer and an enteric polymer.

WO 2007/105229 (Panacea BiotecLtd) provides controlled release pharmaceutical composition comprising at least one active ingredient (e.g. lamotrigine), at least one pH dependent polymer(s), at least one pH independent polymer(s), and at least one channel forming agent(s) and optionally with other pharmaceutically acceptable excipients (e.g. hydration inhibitor), wherein the ratio of the pH independent polymer and the pH independent polymer is 1:10 to 10:1.

US 4,968,508 (Eli Lilly And Company) discloses a matrix formulation comprising cefaclor, hydrophilic polymer, acrylic polymer which dissolves at a pH in the range of between about 5.0 to 7.4.

US 6,074,669 (Ranbaxy Laboratories Limited) is directed to a controlled release formulation comprising hydrophilic polymers, enteric polymer and diltiazem or acceptable salt or ester thereof. The ratio of hydrophilic polymer to enteric polymer is from about 1:1 to about 15:1.

EP 173928 (Ab Leo) discloses controlled release formulation which has a biphasic release profile of a pharmacologically active agent, comprising a drug tablet and a coating applied thereon, wherein the coating essentially consists of a film-forming polymer which is insoluble in water and gastro-intestinal fluids and a water-soluble pore-creating material being randomly distributed in said polymer characterized in that the pore-creating material includes a drug active substance in a therapeutically effective amount.

US 6,514,531 (Sanofi-Synthelabo) discloses controlled release formulation which has a biphasic release profile of zolpidem, where the first phase is an immediate release phase having a maximum duration of 30 minutes and these phases a prolonged release phase, and wherein 40 to 70% of the total amount of zolpidem is release during the immediate release phase and the time for release of 90% of the total amount of zolpidem is between 2 and 6 hours.

In the present case, controlled release formulations of propiverine have been prepared using different techniques other than those discussed in the art to formulate a dosage form which has a feature of biphasic release profile with less than 50% release rate until 9th hour and not less than 85% in 24 hours. There exists a need to design a formulation comprising propiverine which would exhibit acceptable dissolution profile and at the same time would be manufactured by a simple and industrially viable process.

### Summary of the Invention

Based on the prior art, the object of the invention is to develop a pharmaceutical composition comprising propiverine featuring controlled release properties.

It is an object of the present invention to provide a controlled release dosage formulation for propiverine that can be used to prepare a range of dosing levels.

It is a further object of the present invention to provide a controlled release dosage formulation featuring suitable dissolution rates in the specific pH conditions with combinations of pH dependent and hydrophilic polymers in the presence of pore forming agents in different ratios.

It is an additional object of the present invention to obtain a controlled release dosage formulation for propiverine that can provide continuous and therapeutic levels to the patient in need of such treatment for twenty-four (24h) hour period.

More specifically, the present invention relates to an oral dosage form in the form of tablet comprising propiverine or one of its pharmaceutically acceptable salts wherein the release of active ingredient is controlled with pH dependent and hydrophilic polymers in the presence of pore forming agents.

The present invention features biphasic release profile with less than 50% release rate until 9^{th} hour and not less than 85% in 24 hours.

More specifically, the main object of the invention is to develop a pharmaceutical composition for oral administration comprising propiverine such that the said composition exhibits a biphasic *in vitro* release of propiverine characterized as less than 50.0% propiverine release after nine hours and more than 85.0% propiverine release after 24 hours as measured by USP Type I apparatus (basket), at 100 rpm, in 750 mL of 0.1 N hydrochloric acid for the first hour, subsequently in 750 mL USP buffer at pH 5.8 until nine hours, and subsequently in 750 mL of pH 6.8 USP buffer until 24 hours.

### Detailed Description of the Invention

A controlled release dosage formulation for propiverine developed to fulfill the objectives of the present invention is illustrated in the accompanying figures wherein;
Figure 1 shows the dissolution profiles for Formula-1 and Formula-2.
Figure 2 shows the dissolution profiles for Formula-3, 4, 5 and 6.

In the scope of the present invention, although propiverine, being a BCS Class III drug, shows high solubility independent of the pH of the aqueous media, the release properties of the formulation have been modified by using various polymers having different pH dependency.

The general aspect of the present invention provides an oral pharmaceutical composition comprising propiverine or pharmaceutically acceptable salt thereof having biphasic *in vitro* release characteristics, which includes at least one hydrophilic polymer, and at least one water soluble pore forming agent, and at least two pH dependent polymers comprising a mixture.

One aspect of the present invention provides an oral pharmaceutical composition comprising propiverine or pharmaceutically acceptable salt thereof having biphasic *in vitro* release characteristics wherein a dissolution profile of propiverine less than 50.0% after nine hours and not less than 85.0% in 24 hours as measured by USP Type I apparatus (basket) at 100 rpm, in 750 mL of 0.1 N hydrochloric acid for the first hour, subsequently in 750 mL USP buffer at pH 5.8 until nine hours, and subsequently in 750 mL of pH 6.8 USP buffer until 24 hours wherein said composition also comprises;
**a.** at least one hydrophilic polymer selected from hydroxypropyl cellulose, hydroxypropylmethyl cellulose and combination thereof,
**b.** at least one water soluble pore forming agent is selected from lactose hydrous, lactose anhydrous, lactose monohydrate, lactose spray-dried and any combination thereof, wherein it is in an amount of less than 50% by weight of the composition, and
c. at least two pH dependent polymer comprising a mixture of a pH dependent polymer dissolving at or above pH 5.5 and a pH dependent polymer dissolving at or above pH 6.0 in an amount of at least 20% by weight of the composition.

The term "biphasic release" as referred to herein is *in vitro* two-stage dissolution profile which is in a biphasic pattern with an initial first-phase release that is less than 50.0% until 9 hours and followed by a second-phase of release that is more than 50.0% after 9 hours and sustained at a level above 85.0% in 24 hours, at different pH conditions, simulating the GI tract conditions.

For purposes of the present invention the term "controlled release" refers to a pharmaceutical dosage form which releases active ingredient(s) over a prolonged period of time, in this case over a period up to 24 hours, which also be referred to as "prolonged release", "extended release", "modified release", "delayed release" and "sustained release". When used in association with the dissolution profiles discussed herein, the term "controlled release" refers to that portion of a dosage form made according to the present invention which delivers active ingredient(s) over a period of time, at least up to 24 hour. Preferably the present invention is a controlled release oral pharmaceutical formulation and provides a release of the drug at least up to 24 hour.

Thus there is provided a pharmaceutical controlled-release dosage form adapted to release propiverine or a salt thereof over a predetermined time period, according to a biphasic *in vitro* profile of dissolution. The present formulation exhibits the following *in vitro* dissolution profile when measured by USP Type I apparatus (basket), at 100 rpm, in 750 mL of 0.1 N hydrochloric acid for the first hour, subsequently in 750 mL USP buffer at pH 5.8 after nine hours, and subsequently in 750 mL of pH 6.8 USP buffer after 24 hours: a) less than 50.0% release after nine hours and b) not less than 85.0% release in 24 hours.

In the present invention, intended dissolution rates in the specific pH conditions were achieved with combination of at least two pH dependent polymers, at least one pore forming agent and at least one hydrophilic as claimed. During dissolution, the water enters to the tablet through soluble pore-forming excipients as lactose monohydrate and water soluble polymers tend to thicken to form gel as they absorb the water. The forming gel enables the tablet to release propiverine in a slower release. In the said invention water soluble pore forming excipients play a significant role for gel forming as well as gel forming pH independent polymers.

In the controlled release propiverine formulations of the present invention, at least one hydrophilic polymer, at least two pH dependent polymer and at least one water soluble pore forming agents are crucial for the dissolution release profile.

In the controlled release propiverine formulations of the present invention, amount of propiverine or the corresponding equivalent amount of propiverine salt or a mixture thereof is in the range of from 4 mg to 60 mg.

The hydrophilic polymer is selected from hydroxypropyl cellulose, hydroxypropylmethyl cellulose and combination thereof.

The pharmaceutically acceptable pore forming agent is water soluble. The water soluble material present in the coating which dissolves and forms pores in the coating layer may act as pore forming agent. The water soluble pore forming agent is lactose (hydrous, anhydrous, monohydrate, spray dried).

The amount of the pore forming agent in the dosage form is less than 50% by weight of the composition. Preferably, the amount of the pore forming agent may vary within a range of from 10% to 50% by weight of the composition.

In the controlled release propiverine formulations of the present invention, it is found out that the solubility features and the ratios of the two pH dependent polymers play a significant role during the release of propiverine in different conditions.

The present invention comprises pH dependent polymers that are soluble at various, or different in the case of more than one pH. The said formulation comprises pH dependent polymers to achieve a reduced release in lower pH values. The pH dependent polymers stay stable in the low pH values while in the higher pH values, start to dissolve and release propiverine to the dissolution medium. The pH dependency of the polymers and their ratios has significant effect on the attainment of required release characteristics in the modified release pharmaceutical formulations, which enable control of drug release over 24 hours. In order to realize suitable oral dosage forms with a sufficiently prolonged release of the active agent and a therapeutically effective blood level for an interval of 24 hours one has to take into account that such formulations naturally release substantial portions of its content of active agent in the intestine regions.

Roughly, the pH of the duodenum is about 5.5, the jejunum is about 6.5 and the distal ileum is about 7.5. The present invention may control the drug release in GI tract.

In one embodiment of the invention, the pharmaceutical composition comprises at least two pH dependent polymers comprising a mixture of at least one pH dependent polymer dissolving at or above pH 5.5 and at least one pH dependent polymer dissolving at or above pH 6.0.

The pH dependent polymer may be selected from the group of cellulose acetate, cellulose acetate phthalate, cellulose acetate trimellitate, cellulose acetate succinate, hydroxypropyl methylcellulose (hypromellose) phthalate, hydroxypropyl phthalate, hydroxypropyl methyl cellulose acetate phthalate, hydroxypropyl methylcellulose (hypromellose) acetate succinate, polyvinyl acetate phthalate, shellac, co-polymerized methacrylic acid/methacrylic acid methyl esters such as, materials known under the trade name EUDRAGIT® L12.5, L100, L30D-55, L100-55, S100, S12.5 and derivatives and mixtures thereof.

EUDRAGIT® L100 is described at the Ph.Eur (Methacrylic Acid - Methyl Methacrylate Copolymer (1:1)), USP/NF (Methacrylic Acid Copolymer, Type A - NF) and JP (Methacrylic Acid Copolymer L) monographs. Average molar mass is approx. 125,000 g/mol and glass transition temperature (Tg) is above 130°C (+/- 5°C).

Chemical/IUPAC name of L100 is "Poly(methacylic acid-co-methyl methacrylate) 1:1". It dissolves above pH 6.0 and targeted drug release area is jejunum.

EUDRAGIT® L100-55 is described at the Ph.Eur (Methacrylic Acid - Ethyl Acrylate Copolymer (1:1) Type A), USP/NF (Methacrylic Acid Copolymer, Type C - NF) and JP (Dried Methacrylic Acid Copolymer LD) monographs. Average molar mass is approx. 320,000 g/mol and glass transition temperature (Tg) is 96°C (+/- 5°C).

Chemical/IUPAC name of L100-55 is "Poly(methacylic acid-co-ethyl acrylate) 1:1". It dissolves above pH 5.5 and targeted drug release area is duodenum.

Preferably, pH dependent polymer soluble starting from pH 5.5 is selected from, but are not limited to, hypromellose phthalate, hypromellose acetate succinate, cellulose acetate phthalate, copolymer of methylmethacrylic acid and methyl methacrylate; more preferably, but not limited to, Eudragit® L100-55, Eudragit® L30D-55, Kollicoat® MEA 30DP and Kollicoat® MEA 100 P and ESTACRYL® 30D (Eastman Chemical), Shin-Etsu® Hypromellose Phthalate NF-55, Shin-Etsu® Hypromellose Acetate Succinate NF AS-LF and mixtures thereof.

Preferably, pH dependent polymer soluble starting from pH 6.0 is selected from, but not limited to, copolymer of methylmethacrylic acid and methyl methacrylate, cellulose acetate phtalate, cellulose acetate succinate, hydroxypropyl methylcellulose phthalate, hydroxypropyl methylcellulose acetate succinate; more preferably, but not limited Eudragit® L100, Shin-Etsu AQOAT® AS-MF, Shin-Etsu AQOAT® AS-MG and mixtures thereof.

The present invention includes at least two pH dependent polymer comprising a mixture of at least one pH dependent polymer soluble starting from pH 5.5 and at least one pH dependent polymer soluble starting from pH 6.0, and the amount of the mixture in the dosage form is at least 20% by weight of the composition. In a more preferred embodiment, the present invention discloses a suitable weight ratio of pH dependent polymers that the mixture includes pH dependent polymer soluble starting from pH 5.5 in an amount of 4% to 25% by weight of the composition and a pH dependent polymer soluble starting from pH 6.0 in an amount of 10% to 50% by weight of the composition, the present invention discloses suitable weight ratio of pH dependent polymers.

The said invention is achieved by combining propiverine or one of its pharmaceutically acceptable salts with at least two pH dependent polymers, at least one hydrophilic polymer and at least one water soluble pore forming agent may further include one or more of other pharmaceutically acceptable excipients selected from, but not limited to, binder, lubricant, diluents, flavoring agent, sweetener, coloring agent, surfactant, stabilizing agent, viscosity agent, film coating agents or any combination thereof.

In one embodiment of the invention, the pharmaceutical composition comprises at least one lubricant, selected from the group consisting of calcium stearate, glycerine monostearate, magnesium stearate, stearic acid, glyceryl palmitostearate, glyceryl behenate, hydrogenated castor oil, hydrogenated vegetable oil, sodium lauryl sulphate, magnesium lauryl sulphate, sodium stearyl fumarate, poloxamer, polyethylene glycol, sucrose ester of fatty acids, talc and any combination thereof.

In one embodiment of the invention, the pharmaceutical composition comprises at least one diluent/filler, selected from the group consisting of calcium carbonate, dibasic calcium phosphate anhydrous, calcium phosphate dihydrate, tribasic calcium phosphate, calcium sulphate, cellulose powdered, microcrystalline cellulose, silicified microcrystaline cellulose, cellulose acetate, colloidal silicon dioxide, dextrin, dextrose, lactose, magnesium carbonate, maltodextrin, mannitol, xylitol, polydextrose, starch, pregelatinized starch, calcium phosphate and any combination thereof.

In another preferred embodiment, the pharmaceutical composition comprises at least one binder, selected from the group consisting of carbomer, carboxymethylcellulose sodium, dextrin, dextrose, maltodextrin, gelatin, hydroxyethyl cellulose, hydroxyethylmethyl cellulose, hydroxypropyl cellulose, low substituted hydroxypropyl cellulose, ethylcellulose, methyl cellulose, hypromellose, magnesium aluminium silicate, methylcellulose, povidone, sodium alginate, starch, pregelatinised starch, liquid glucose, sucrose, tragacanth (gum benjamin), zein, acacia (gum arabic), alginic acid, guar gum, copovidone and any combination thereof.

Preferably, at least one sweetening agent is selected from the group consisting of aspartame, acesulfame potassium, saccharin sodium, cyclamates, sucralose, corn syrup, sorbitol (solution), maltitol (syrup), oligosaccharide, isomaltooligosaccharide, fructose, lactose, glucose, lycasin, xylitol, lactitol, erythritol, mannitol, isomaltose, polydextrose, dextrin or mixtures thereof.

In one embodiment of the invention, at least one flavoring agent is selected from the group consisting of natural and/or semi-synthetic or synthetic flavoring agents such as citrus, lemon, lime, orange, tangerine, grape fruit, peppermint, spearmint, apple, pear, plum, peach, apricot, strawberry, raspberry, cherry, banana, vanilla, watermelon, honey, bubble gum flavor or mixtures thereof.

In one embodiment of the invention, film coating agents include but not limited to hydroxypropylmethyl cellulose, ethyl cellulose, cellulose acetate, polyvinyl alcohol, acrylic and/or methacrylic co-polymers, resins, one or more plastifying agents (e.g., polyethylene glycol, propylene glycol, glycerin, triethyl citrate, diethyl phthalate, and mixtures thereof.

In one embodiment of the invention, viscosity agents include but not limited to carboxymethyl cellulose sodium, carboxymethyl cellulose calcium, chitosan, colloidal silicon dioxide, gelatin, guar gum, xanthan gum, hydroxyethyl cellulose, hydroxypropyl cellulose, hydroxymethyl cellulose, hypromellose, maltodextrine, polyvinyl alcohol and the like or combinations thereof.

In one embodiment of the invention, stabilizing agents include, but not limited to, antioxidants, chelating agent, photoprotectant and alkalizing agent or mixtures thereof.

Preferably, surfactants include, but not limited, to alkyl benzene sulfones, alkyl sulfates, ether carboxylates, glycerol/propylene glycol fatty acid esters, hexadecyl triammonium bromide, hydroxylated lecithin, lauryl carnitine, lower alcohol-fatty acid esters, mono/diglycerides, polyethylene glycol alkyl ethers, polyethylene glycol-fatty acid monoesters, polyethylene glycol-fatty acid diesters, polyethylene glycol-glycerol esters, polyethylene glycol phenols, polyethylene glycol-sorbitan fatty acid esters, polyglyceride fatty acids, polyoxyethylene-polyoxypropylene block copolymers, propylene glycol-fatty acid esters, sodium cholate, sodium lauryl sulfate, sodium palmitate, sodium taurocholate, sorbitanfatty acid esters, sterol and sterol derivatives, sugar esters, transesterification products of oils and alcohols and mixtures thereof.

In one embodiment, the present invention is a pharmaceutical composition which is suitable for oral administration, such as tablet, film-coated tablet, dispersible tablet, orally disintegrating tablet, powder, granules or a combination thereof. Preferred dosage form is tablet. In a more preferred embodiment of the invention, the said pharmaceutical composition is formulated in a single dosage form for oral administration such as tablet or film-coated tablet.

Another embodiment discloses a process of manufacturing controlled release formulation of propiverine or a pharmaceutically acceptable salt thereof. The tablet formulation may be produced with the techniques of direct compression, dry granulation and wet granulation. In the preferred embodiment of the present invention, direct compression method is applicable as well as wet granulation and dry granulation techniques. Water, ethanol, isopropyl alcohol and mixtures thereof can be used as solvents during wet granulation.

Direct compression technique has the least steps for tablet manufacturing and more advantageous for the industry therefore direct compression technique is usually the first approach while developing a new pharmaceutical formulation. Granulation techniques are applied if the powder properties of direct compression powder blend as powder flow, compressibility, homogeneity etc. are problematic. Said pharmaceutical invention is advantageous for the industry since formulation blends are very suitable for direct compression technique.

Said pharmaceutical composition is compressed into tablets after direct compression or dry granulation or wet granulation. The tablets may be film-coated in a conventional manner.

The controlled release formulation may also include one or more additional active ingredients.

Having described the invention with reference to certain embodiments, other embodiments will become apparent to the person skilled in the art from consideration of the specification. Certain specific aspects and embodiments of the invention will be further described in the following examples, which are provided solely for purposes of illustration and are not intended to limit the scope of the invention in any manner.

### Preparation of the composition

The composition may be produced in conventional manner by mixing the components. Below are examples of specific forms of the composition allowing various release profile of the active agent contained therein. All the excipients in the below compositions are mixed, sieved and compressed into tablets with the appropriate tablet weights.

### Examples

### Example 1 - Dissolution Method

The dissolution conditions were designated considering the gastrointestinal pathway of orally applied solid dosage forms. The first pH condition in the dissolution method designated is similar to stomach as pH 1.2 and pH 5.8 and 6.8 conditions are chosen with respect to duodenum, jejunum and ileum regions of the GI tract.

The tablets were tested for dissolution using the procedure demonstrated in Table 1, with the formula set forth in Table 2.

**Table 1. Dissolution method**

| | | |
|---|---|---|
| **Equipment:** | HPLC | |
| **Wavelength:** | 220 nm | |
| **Apparatus:** | USP type I (basket) | |
| **Rotation speed:** | 100 rpm | |
| **Buffers:** | 1^{st} stage | 0-1 hour, 0.1N hydrochloric acid, from 1 to 9 hours, pH 5.8 USP buffer |
| | 2^{nd} stage | From 9 to 24 hours, pH 6.8 USP buffer |
| **Buffer volume:** | 750 mL | |
| **Temperature:** | 37°C± 0.5°C | |

**Table 2. Tablet Formulations**

| | Formula-1 | Formula-2 | Formula-3 | Formula-4 | Formula-5 | Formula-6 |
|---|---|---|---|---|---|---|
| Ingredients | % | % | % | % | % | % |
| Propiverine Hydrochloride | 5.6 | 5.6 | 5.6 | 5.6 | 5.6 | 5.6 |
| Lactose Monohydrate Spray Dried | 55.9 | 59.7 | 32.8 | 39.1 | 45.3 | 37.8 |
| Hydroxypropyl Cellulose | 3.1 | 3.1 | 3.1 | 3.1 | 3.1 | 3.1 |
| Hydroxypropyl Methyl Cellulose K100LV | 12.5 | 12.5 | 12.5 | 12.5 | 12.5 | 12.5 |
| Hydroxypropyl Methyl Cellulose K4M | 5.6 | 5.6 | 5.6 | 5.6 | 5.6 | 5.6 |
| Eudragit®L 100 | 9.4 | 6.3 | 20.0 | 24.4 | 20.0 | 25.0 |
| Eudragit®L 100-55 | 6.9 | 6.3 | 19.4 | 8.8 | 6.9 | 9.4 |
| Magnesium Stearate | 1.0 | 1.0 | 1.0 | 1.0 | 1.0 | 1.0 |

The suitable formulations with reference to certain embodiments provide a biphasic dissolution of propiverine less than 50.0% after nine hours and not less than 85.0% in 24 hours as measured by USP Type I apparatus (basket) at 100 rpm, in 750 mL of 0.1N hydrochloric acid for the first hour, subsequently in 750 mL USP buffer at pH 5.8 until nine hours, and subsequently in 750 mL of pH 6.8 USP buffer until 24 hours

The dissolution results are set forth in Table 3 below:

**Table 3. Dissolution results of tablet formulations referred in Table 2.**

| Time/Condition | Formula-1 % release | Formula-2 % release | Formula-3 % release | Formula-4 % release | Formula-5 % release | Formula-6 % release |
|---|---|---|---|---|---|---|
| 1^{st}hour/HCl | 19,33 | 27,19 | 22,31 | 15,60 | 21,29 | 14,72 |
| 3^{rd} hour / pH:5.8 | 34,03 | 48,89 | 29,93 | 27,61 | 34,62 | 21,76 |
| 5^{th} hour / pH:5.8 | 47,05 | 60,00 | 31,51 | 33,96 | 40,47 | 26,18 |
| 7^{th} hour/ pH:5.8 | 58,38 | 64,57 | 33,33 | 38,23 | 44,57 | 30,24 |
| 9^{th} hour / pH:5.8 | 68,56 | 70,20 | 34,46 | 41,14 | 47,46 | 33,90 |
| 12^{th} hour / pH:6.8 | 79,88 | 80,90 | 56,35 | 66,43 | 76,92 | 59,11 |
| 24^{th} hour / pH:6.8 | 90,38 | 93,10 | 93,71 | 85,52 | 93,52 | 89,92 |

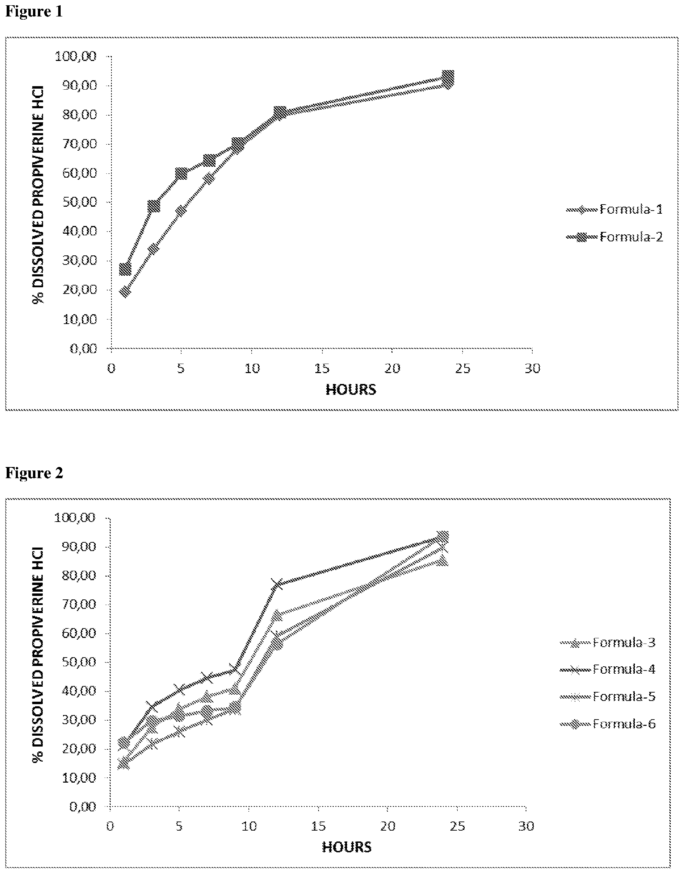

The examples of the controlled release propiverine formulations of Formula-1 and Formula-2 in Figure 1 display dissolution rates above 50% at 9th hour. The said two formulations comprise the pH dependent polymers (EUDRAGIT® L 100 and EUDRAGIT® L 100-55) and pore forming agent (Lactose Monohydrate Spray Dried) out of the limits, herein the amount of the mixture of the two pH dependent polymers is less than 20% by weight and the amount of pore forming agent is more than 50% by weight of the composition (Figure 1).

The examples of the controlled release propiverine formulations of Formula-3, Formula-4, Formula-5 and Formula-6 which comprise the pH dependent polymers and pore forming agent in the limits of the present invention have suitable dissolution profiles regarding the desire limits of the present invention (Figure 2).

While the invention has been described with respect to the above specific embodiments, it should be recognized that various modifications and changes may be made to the invention by those skilled in the art which also fall within the scope of the invention as defined by the appended claims.

## Claims

1. An oral pharmaceutical composition comprising propiverine or pharmaceutically acceptable salt thereof having biphasic *in vitro* release characteristics wherein a dissolution profile of propiverine less than 50.0% after nine hours and not less than 85.0% in 24 hours as measured by USP Type I apparatus (basket) at 100 rpm, in 750 mL of 0.1 N hydrochloric acid for the first hour, subsequently in 750 mL USP buffer at pH 5.8 until nine hours, and subsequently in 750 mL of pH 6.8 USP buffer until 24 hours wherein said composition comprises
**a.** at least one hydrophilic polymer selected from hydroxypropyl cellulose, hydroxypropylmethyl cellulose and combination thereof,
**b.** at least one water soluble pore forming agent selected from lactose hydrous, lactose anhydrous, lactose monohydrate, lactose spray-dried and any combination thereof, wherein it is in an amount of less than 50% by weight of the composition, and
**c.** at least two pH dependent polymer comprising a mixture of a pH dependent polymer dissolving at or above pH 5.5 and a pH dependent polymer dissolving at or above pH 6.0 in an amount of at least 20% by weight of the composition.

2. An oral pharmaceutical composition according to claim 1, wherein the water soluble forming agent is within a range of from 10% to 50% by weight of the composition.

3. An oral pharmaceutical composition according to claim 1 wherein the pH dependent polymer soluble starting from pH 5.5 is selected from the group consisting of hypromellose phthalate, hypromellose acetate succinate, cellulose acetate phthalate, copolymer of methylmethacrylic acid and methyl methacrylate; and any combination thereof.

4. An oral pharmaceutical composition according to claim 1 wherein the pH dependent polymer soluble starting from pH 6.0 is selected from the group consisting of copolymer of methylmethacrylic acid and methyl methacrylate, cellulose acetate phtalate, cellulose acetate succinate, hydroxypropyl methylcellulose phthalate, hydroxypropyl methylcellulose acetate succinate; and any combination thereof.

5. An oral pharmaceutical composition according to claim 1, wherein the mixture comprises a pH dependent polymer soluble starting from pH 5.5 in an amount of 4% to 25% by weight of the composition and a pH dependent polymer soluble starting from pH 6.0 in an amount of 10% to 50% by weight of the composition.

6. An oral pharmaceutical composition according to any of the preceding claims, wherein the composition further comprises one or more additional excipients.

7. An oral pharmaceutical composition according to claim 8 wherein the dosage form is tablet or film-coated tablet.

8. An oral pharmaceutical composition according to any of the preceding claims, wherein an amount of 4 mg to 60 mg of propiverine or the corresponding equivalent amount of propiverine salt or a mixture thereof is included.

9. An oral pharmaceutical composition according to claim 10 wherein the composition comprises propiverine hydrochloride.

10. An oral pharmaceutical composition according to any of the preceding claims, for use in the treatment of overactive bladder and urinary incontinence.

## Patentansprüche

1. Orale pharmazeutische Zusammensetzung, umfassend Propiverin oder ein pharmazeutisch annehmbares Salz davon mit biphasischen in-vitro-Freisetzungseigenschaften, wobei ein Auflösungsprofil von Propiverin von weniger als 50,0% nach neun Stunden und nicht weniger als 85,0 % in 24 Stunden, gemessen mit einer USP-Typ-I-Apparatur (Korb) bei 100 rpm/min, in 750 mL 0,1N Salzsäure für die erste Stunde, anschließend in 750 mL USP-Puffer bei pH 5,8 bis zu neun Stunden und anschließend in 750 mL pH 6,8 USP-Puffer bis zu 24 Stunden, wobei die Zusammensetzung umfaßt
**a.** mindestens ein hydrophiles Polymer, ausgewählt aus Hydroxypropylcellulose, Hydroxypropylmethylcellulose und deren Kombination,
**b.** mindestens ein wasserlösliches porenbildendes Mittel, ausgewählt aus wasserhaltiger Laktose, wasserfreier Laktose, Laktosemonohydrat, sprühgetrockneter Laktose und jeder Kombination davon, wobei es in einer Menge von weniger als 50 Gew.-% der Zusammensetzung vorliegt, und
**c.** mindestens zwei pH-abhängige Polymere, umfassend eine Mischung aus einem pH-abhängigen Polymer, das sich bei oder über pH 5,5 auflöst, und einem pH-abhängigen Polymer, das sich bei oder über pH 6,0 auflöst, in einer Menge von mindestens 20 Gew.-% der Zusammensetzung

2. Orale pharmazeutische Zusammensetzung nach Anspruch 1, wobei das wasserlösliche Bildungsmittel in einem Bereich von 10 bis 50 Gew.-% der Zusammensetzung liegt.

3. Orale pharmazeutische Zusammensetzung nach Anspruch 1, wobei das pH-abhängige Polymer, das ab pH 5,5 löslich ist, ausgewählt ist aus der Gruppe bestehend aus Hypromellosephthalat, Hypromelloseacetatsuccinat, Celluloseacetatphthalat, Copolymer aus Methylmethacrylsäure und Methylmethacrylat; und jeder Kombination davon.

4. Orale pharmazeutische Zusammensetzung nach Anspruch 1, wobei das pH-abhängige Polymer, das ab pH 6,0 löslich ist, ausgewählt ist aus der Gruppe bestehend aus Copolymer von Methylmethacrylsäure und Methylmethacrylat, Celluloseacetatphthalat, Celluloseacetatsuccinat, Hydroxypropylmethylcellulosephthalat, Hydroxypropylmethylcelluloseacetatsuccinat; und jeder Kombination davon.

5. Orale pharmazeutische Zusammensetzung nach Anspruch 1, wobei die Mischung ein pH-abhängiges Polymer, das ab pH 5,5 löslich ist, in einer Menge von 4 bis 25 Gew.-% der Zusammensetzung und ein pH-abhängiges Polymer, das ab pH 6,0 löslich ist, in einer Menge von 10 bis 50 Gew.-% der Zusammensetzung umfasst.

6. Orale pharmazeutische Zusammensetzung nach einem der vorhergehenden Ansprüche, wobei die Zusammensetzung weiterhin einen oder mehrere zusätzliche Hilfsstoffe umfasst.

7. Orale pharmazeutische Zusammensetzung nach Anspruch 8, wobei die Darreichungsform eine Tablette oder eine Filmtablette ist.

8. Orale pharmazeutische Zusammensetzung nach einem der vorhergehenden Ansprüche, wobei eine Menge von 4 mg bis 60 mg Propiverin oder die entsprechende äquivalente Menge eines Propiverinsalzes oder eine Mischung davon enthalten ist.

9. Orale pharmazeutische Zusammensetzung nach Anspruch 10, wobei die Zusammensetzung Propiverinhydrochlorid umfasst.

10. Orale pharmazeutische Zusammensetzung nach einem der vorhergehenden Ansprüche, zur Verwendung bei der Behandlung von überaktiver Blase und Harninkontinenz.

## Revendications

1. Composition pharmaceutique orale comprenant de la propiverine ou un sel pharmaceutiquement acceptable de celle-ci ayant des caractéristiques de libération in vitro biphasiques, dans laquelle un profil de dissolution de la propiverine inférieur à 50,0 % après neuf heures et non inférieur à 85,0 % en 24 heures, tel que mesuré par un appareil USP de type I (panier) à 100 tours/minute, dans 750 ml d'acide chlorhydrique 0,1 N pendant la première heure, puis dans 750 ml de tampon USP à pH 5,8 jusqu'à neuf heures, et ensuite dans 750 ml de tampon USP à pH 6,8 jusqu'à 24 heures, dans laquelle ladite composition comprend
**a.** au moins un polymère hydrophile choisi parmi l'hydroxypropylcellulose, l'hydroxypropylméthylcellulose et leur combinaison,
**b.** au moins un agent porogène hydrosoluble choisi parmi le lactose hydraté, le lactose anhydre, le lactose monohydraté, le lactose séché par pulvérisation et toute combinaison de ceux-ci, dans lequel il est présent en une quantité inférieure à 50 % en poids de la composition, et
**c.** au moins deux polymères dépendant du pH comprenant un mélange d'un polymère dépendant du pH se dissolvant à un pH égal ou supérieur à 5,5 et d'un polymère dépendant du pH se dissolvant à un pH égal ou supérieur à 6,0 en une quantité d'au moins 20 % en poids de la composition

2. Composition pharmaceutique orale selon la revendication 1, dans laquelle l'agent formateur soluble dans l'eau est dans une gamme de 10 % à 50 % en poids de la composition.

3. Composition pharmaceutique orale selon la revendication 1, dans laquelle le polymère dépendant du pH, soluble à partir de pH 5,5, est choisi dans le groupe constitué par le phtalate d'hypromellose, l'acétate succinate d'hypromellose, l'acétate phtalate de cellulose, le copolymère d'acide méthyl méthacrylique et de méthacrylate de méthyle ; et toute combinaison de ceux-ci.

4. Composition pharmaceutique orale selon la revendication 1, dans laquelle le polymère dépendant du pH, soluble à partir de pH 6,0, est choisi dans le groupe constitué par un copolymère d'acide méthyl méthacrylique et de méthacrylate de méthyle, un phtalate d'acétate de cellulose, un succinate d'acétate de cellulose, un phtalate d'hydroxypropylméthylcellulose, un succinate d'acétate d'hydroxypropylméthylcellulose ; et toute combinaison de ceux-ci.

5. Composition pharmaceutique orale selon la revendication 1, dans laquelle le mélange comprend un polymère dépendant du pH soluble à partir de pH 5,5 en une quantité de 4% à 25% en poids de la composition et un polymère dépendant du pH soluble à partir de pH 6,0 en une quantité de 10% à 50% en poids de la composition.

6. Composition pharmaceutique orale selon l'une quelconque des revendications précédentes, dans laquelle la composition comprend en outre un ou plusieurs excipients supplémentaires.

7. Composition pharmaceutique orale selon la revendication 8, dans laquelle la forme posologique est un comprimé ou un comprimé pelliculé.

8. Composition pharmaceutique orale selon l'une quelconque des revendications précédentes, dans laquelle est incluse une quantité de 4 mg à 60 mg de propiverine ou la quantité équivalente correspondante de sel de propiverine ou un mélange de ceux-ci.

9. Composition pharmaceutique orale selon la revendication 10, dans laquelle la composition comprend du chlorhydrate de propiverine.

10. Composition pharmaceutique orale selon l'une quelconque des revendications précédentes, destinée à être utilisée dans le traitement de la vessie hyperactive et de l'incontinence urinaire.
